# EUROPEAN PATENT APPLICATION

(11) **EP 0 561 758 A2**
(43) Date of publication of application: **22.09.1993**
(21) Application number: 93870046.5
(22) Date of filing: 15.03.1993
(51) Int. Cl.: C07C 227/30, C07C 271/22, C07C 271/34, C07C 271/54, C07C 227/32

(54) **Method of preparing optically active homo-beta-amino acids**

(30) Priority: 18.03.1992 US 853561
(71) Applicant: MONSANTO COMPANY, St. Louis Missouri 63167 (US)
(72) Inventor: Talley, John Jeffrey, St Louis, Missouri 63132 (US)
(74) Representative: Ernst, Hubert

(57) **Abstract**

The present invention is directed to synthesis of homo-β-amino acids of an optical purity sufficient to exhibit optical activity wherein Curtius rearrangement of 3-mono-substituted succinate acid half ester of an optical purity sufficient to exhibit optical activity is affected and the incipient isocyanate is trapped with a primary or secondary alcohol. The resulting carbamate-protected homo-β-amino esters are then saponified to produce the corresponding carbamate-protected homo-β-amino acids which are deprotected to yield homo-β-amino acids of an optical purity sufficient to exhibit optical activity.

## Description

### BACKGROUND OF THE INVENTION

### 1. Field of the Invention

The present invention relates to synthesis of homo-β-amino acids of an optical purity sufficient to exhibit an optical activity and, more particularly, relates to a method of preparing optically active homo-β-amino acids wherein Curtius rearrangement of a particular class of chiral mono-substituted succinic acid esters and trapping of the incipient isocyanate derivative with a primary or secondary alcohol produces carbamate-protected homo-β-amino esters of a purity sufficient to exhibit optical activity in high yield which are subsequently saponified and deprotected to yield the corresponding optically active homo-β-amino acid of the same chirality as the starting succinate.

### 2. Related Art

Homo-β-amino acids are useful replacements in pharmacologically active peptides for the corresponding α-amino acid in order to potentiate the activity of the peptide or to increase resistance thereof to enzymatic degradation. For example, [1-β-alanine]-ACTH (1-18) - octadecapeptide has enhanced and prolonged adrenocorticotropic and lipotropic activities. In addition, substitution of β-amino acids within certain bradykinins have been shown to be resistant to dipeptidylcarboxypeptidase and a number of vasopressin analogues have been made with β-amino acid residues.

Various methods have been proposed for the preparation of optically active β-amino acids. See, for example, *Chemistry and Biochemistry of Amino Acids,* Vol. 4, Chapter 5, pp 250-57, B. Weinstein, Ed., Dekker, N.Y. (1975). Furukawa et al, Chem. Pharm. Bull., 25, 1319 (1977), disclose asymmetric synthesis of β-amino acids by addition of chiral amines to carbon-carbon double bonds having nitrile or ester groups in the α-position. β-amino acids have been produced with optical purities ranging from 2 to 28% by reacting chiral Schiff bases with Reformsky reagent. Terentev et al, Dohl. Ahad. Nauh SSSR, 163,674 (1965) disclose synthesis of β-aminobutyric acids involving addition of chiral amines to crotonic acid with optical purities ranging from 7-9%.

Brown et al, Tetrahedron Lett., Vol. 28, No. 19, pp 2179-2182 (1987), disclose a method of preparing optically active disubstituted β-amino acids which involves asymmetric catalytic hydrogenation of N-substituted α-(aminoalkyl) acrylates. In order to verify the stereochemistry of the product, Curtius rearrangement was effected on the monomethyl ester of optically enriched R,R-anti-2,3-dimethyl-succinic acid and trapping of the incipient isocyanate derivative with a tertiary alcohol, namely, t-butyl alcohol, to give the corresponding R-enriched β-amino acid. Although no yields were reported utilizing t-butyl alcohol, when this Curtius rearrangement was repeated, the yield was only about 23% based on the starting disubstituted succinic acid. Thus, although such method may be useful for verification purposes, from practical commercial standpoint such method is not acceptable.

Ninomiya et al, Tetrahedron Lett., Vol. 30, 2152-2157 (1975) studied the Curtius rearrangement in a process for producing racemic α-amino acids utilizing benzoic acid, diphenylphosphoryl azide (DPPA) and triethylamine followed by treatment with various alcohols and found that t-butyl alcohol gives yields superior to benzyl alcohol, ethanol and phenol.

Ninomiya et al, Chem. Pharm. Bull., Vol. 22, 1398-1404 (1974) also teach that for the production of racemic α-amino acids treatment of monoesters of racemic malonic acid with DPPA/triethylamine in the presence of benzyl alcohol or t-butyl alcohol gives only the benzyl or t-butyl esters and no carbamate. Ninomiya et al teach that the carbamate is formed only when the alcohol is added after heating the acid with DPPA/triethylamine.

In European Patent 0396 526 (published on November 7, 1990), I describe the synthesis of β-amino acids wherein Curtius rearrangement of 2(R)-mono-substituted succinates is effected and the incipient isocyanate is trapped with a primary or secondary alcohol. The resulting carbamate-protected β-amino esters then are saponified to produce the corresponding carbamate-protected β-amino acids which then can be deprotected to produce β-amino acids having the same absolute configuration as naturally-occurring (L)-amino acids.

The chiral succinates used as the starting materials for β-amino acid synthesis in this prior European patent application differ from the chiral succinates used in accordance with the present invention. In this prior publication, the starting succinic acid half esters were substituted at the carbon atom adjacent to the ester moiety. In the present invention, the starting succinic acid half esters (homo-β) are substituted instead on the carbon atom adjacent to the acid moiety. As indicated more fully below, in light of the prior art existing at the time of the present invention, this difference in structure had a major impact on the expectation one skilled in the art would have had about the applicability of the synthesis process of this prior publication to the starting succinates of the present invention.

Ninomiya et al, Chem. Pharm. Bull., 22(8) 1795-1799 (1974), in particular, discuss the Curtius rearrangement of malonic acid half esters with DPPA in the presence of triethylamine. The article teaches that an elimination reaction occurs involving the moiety produced upon reaction between DPPA and the carboxyl (acid) moiety of the malonic acid half ester and a hydrogen atom on the adjacent carbon atom to produce a ketene intermediate. The formation of the ketene unavoidably results in loss of the hydrogen and thus a loss in any chirality of the carbon atom adjacent to the acid moiety, which in turn produces a loss in any optical activity of the parent half ester via such non-chiral ketene intermediate.

### BRIEF SUMMARY OF THE INVENTION

It has now been discovered, contrary to the Ninomiya et al teachings, that elimination of a hydrogen and the formation of a non-chiral ketene intermediate is not a problem when subjecting 3-mono-substituted succinic acid half esters to Curtius rearrangement. Thus, homo-β-amino acids of a purity sufficient to exhibit optical activity can be synthesized from optically active 3-mono-substituted succinic acid half esters using a primary or secondary alcohol to trap the isocyanate derivative of the optically active mono-substituted succinate.

Accordingly, the present invention is directed to synthesis of chiral homo-β-amino acids of a high optical purity wherein Curtius rearrangement of chiral 3-mono-substituted succinates (i.e. succinic acid half esters) is effected and the incipient isocyanate is trapped with a primary or secondary alcohol. The resulting carbamate-protected homo-β-amino esters are then saponified to produce the corresponding carbamate-protected homo-β-amino acids which are then deprotected to produce homo-β-amino acids, retaining the same absolute configuration as the starting succinic acid half ester.

The overall reaction sequence can be shown as follows: 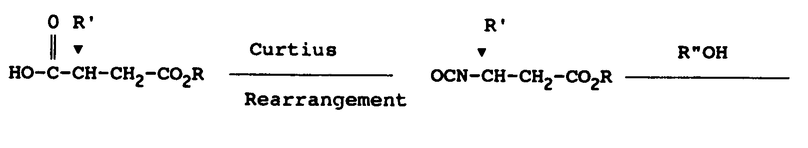 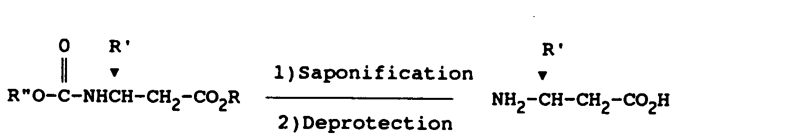
wherein R and R¹ independently represent substituted and unsubstituted alkyl radicals having from 1 to about 12 carbon atoms, substituted and unsubstituted cycloalkyl radicals having from about 4 to about 7 carbon atoms, and substituted and unsubstituted aryl, aralkyl and alkaryl radicals, and R'' represents radicals derived from primary and secondary alcohols.

### DETAILED DESCRIPTION OF THE INVENTION

This invention is directed to asymmetric synthesis of homo-β-amino acids such as represented by the formula: 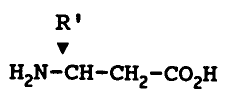
wherein R' represents radicals as defined above.

The subject method for preparing such compounds involves Curtius rearrangement of chiral 3-mono-substituted succinates of a sufficient purity to exhibit optical activity such as represented by the formula: 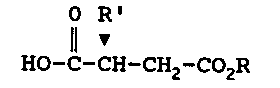
wherein R and R' are the same as defined above, to afford the isocyanate derivative:

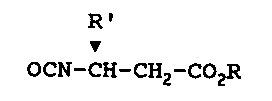

Curtius rearrangement involves pyrolysis of acyl azides acyl azides 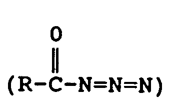
to yield isocyanates (R-N=C=0) which can be subsequently hydrolyzed to give amines. See March, *Advanced Organic Chemistry,* p. 1005, 2nd ed (1977). As a general rule, Curtius rearrangement is a concerted reaction and therefore proceeds with retention of configuration of the starting materials. Determination of specific reaction conditions for effecting Curtius rearrangements of various 3-mono-substituted succinates is within the skill of one in the art familiar with such reactions. In the method of the present invention, Curtius rearrangement to afford the desired isocyanate is preferably effected by treating a chiral 3-mono-substituted succinate (succinic acid half ester) of a high optical purity with one equivalent of diphenoxyphosphoryl azide (PhO)₂PON₃ and triethylamine to form the acyl azide followed by heating in an inert solvent, such as in warm toluene, preferably at about 80° C. for about three hours, to afford the isocyanate derivative.

The 3-mono-substituted succinates can be prepared by a procedure analogous to that described in U.S. 4,939,288 filed January 23, 1989, which is incorporated herein by reference, and as more fully illustrated in the following examples.

The present invention resides in the discoveries that the production of the isocyanate does not proceed through a ketene intermediate which would cause a loss of optical activity and the isocyanate thus produced can be treated directly with a primary or secondary alcohol (R''OH) in the presence of a catalytic amount of a Lewis base such as, for example, dimethylaminopyridine or its equivalent, to afford unexpected high yield of the corresponding carbamate-protected homo-β-amino acid ester represented by the formula:

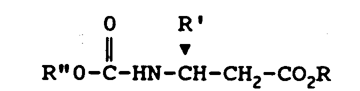

Suitable primary and secondary alcohols include those represented by the formula R''OH wherein R'' represents substituted and unsubstituted alkyl, cycloalkyl and aryl radicals, as well as suitable equivalents such as, for example, silyl radicals. Preferably, the primary and secondary alcohols are those wherein R'' represents substituted and unsubstituted, straight chain as well as branched chain, alkyl radicals having from 1 to about 12 carbon atoms, substituted and unsubstituted cycloalkyl radicals having from 4 to about 7 carbon atoms, and substituted and unsubstituted aryl, alkaryl and aralkyl radicals. Examples of such suitable alcohols include benzyl alcohol, isopropyl alcohol, 4-methoxybenzyl alcohol, 2-trimethylsilylethanol, fluorenyl methanol'and benzhydrol. Preferred alcohols are benzyl alcohol and 4-methoxybenzyl alcohol. Other primary and secondary alcohols suitable for use in the practice of the present invention will be readily apparent to those skilled in the art.

The ester derivative is then saponified by any one of numerous well-known procedures, such as by treatment with aqueous lithium hydroxide/THF (tetrahydrofuran), preferably for three hours at 0° C. The resultant product is the corresponding carbamate-protected homo-β-amino acid represented by the formula: 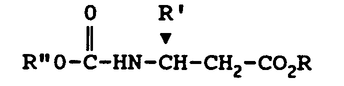
wherein R' and R'' are the same as defined above. These are subsequently deprotected by any one of several well-known procedures, such as by acid catalyzed hydrolysis or by hydrogenolysis (hydrogenation), to produce the corresponding deprotected homo-β-amino acids represented by the formula:

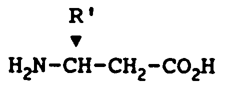

Contemplated equivalents of the general formulas set forth above for the β-amino acids as well as the intermediates are compounds otherwise corresponding thereto and having the same general properties wherein one or more of R, R' and/or R'' are simple variations of the substituents as defined therein, e.g., wherein R' is a higher alkyl group. In addition, where a substituent is designated as, or can be, a hydrogen, the exact chemical nature of a substituent which is other than hydrogen at that position is not critical so long as it does not adversely affect the overall synthesis procedure in terms-of yield.

The chiral center of the optically pure homo-β-amino acids of the present invention, and of the predecessor compounds derived from the chiral monosubstituted succinic acid esters may have either the "R" or "S" configuration, though preferably they have an "R" configuration. The terms "S" and "R" configuration are as defined by the IUPAC 1974 Recommendations for Section E, Fundamental Stererochemistry, Pure Appl. Chem. (1976) 45, 13-30. The process of the present invention preserves the chirality of the starting succinates.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the following examples, melting points were determined on a Fisher-Johns melting point apparatus and are uncorrected. Infrared spectra were measured on an IBM IR30 instrument, absorbance positions are reported in cm-1. Proton and carbon magnetic resonance spectra were recorded on a Varian VXR-300 spectrometer using tetramethylsilane as internal standard. Liquid chromatography was performed on a Spectra Physics chromatography system. All instruments were utilized according to the manufacturer's directions.

### EXAMPLE 1

Preparation of 1-methyl-4-*tert*-butyl-2-(triphenylphosphoranylidene) succinate. A 1L three necked round bottomed flask equipped with a reflux condenser, nitrogen inlet, thermometer adapter and mechanical stirrer was charged with methyl (triphenylphosphoranylidene)acetate (50g, 0.15 mol), *tert*-butyl bromoacetate (15g, 0.08 mol) and 200 mL of ethyl acetate. The mixture was then heated to reflux for 4h. The precipitated methyl (triphenyphosphonium) acetate bromide was removed by filtration and was washed with several portions of ethyl acetate. The filtrate was concentrated in vacuo to produce a tan solid. The crude solid was swirled with two portions of hexane containing a small amount of ethyl acetate to remove the color. This solid material was isolated by filtration and dried in vacuo to give 34.0g, 95%, mp 156-158°C., of the desired Wittig reagent. The NMR spectrum of this material is somewhat unusual in that a 1:1 mixture of conformational isomers is present. ¹H NMR (CDCl₃) 300MHz 7.72-7.62(m, 6H, Ar**H**), 7.56-7.40(m, 9H, Ar**H**), 3.56 & 3.12(s, 3H, OC**H**₃), 2.93(d, J=17.6Hz, 1H, C**H**₂) & 2.79(d, J=17.9Hz, 1H, C**H**₂), 1.32 & 1.27(s, 9H, OC(C**H**₃)₃).

### EXAMPLE 2

Wittig condensation of phenylacetaldehyde with 1-methyl-4-*tert*-butyl-2-(triphenylphosphoranylidene) succinate: Preparation of *mono*-methyl *mono*-*tert*-butyl 2(E)-phenethylidenesuccinate. A 250 mL one necked round bottomed flask equipped with a nitrogen inlet and magnetic stir bar was charged with 1-methyl-4-*tert*-butyl-2-(triphenylphosphoranylidene) succinate (23.9g, 0.053 mol)(Example 1), phenylacetaldehyde (6.24mL, 0.53 mol) and tetrahydrofuran (50 mL). The solution was stirred at room temperature for 6 days and then concentrated on a rotary evaporator to give an oil that was taken up in ethyl acetate whereupon some crystals formed that were removed by filtration and the filtrate concentrated and the residue purified on a Prep-500 on SiO₂ eluting with hexane/ethyl acetate. The pure fractions were combined and concentrated to give 9.70g of pure clear oil, 63%. ¹H NMR (CDCl₃) 300 MHz 7.37-7.20(m, 5H), 7.10(t, J=7.8 Hz, 1H), 3.80(s, 3H), 3.58(d, J=7.8 Hz, 2H), 3.41(s, 2H), 1.50(s, 9H).

### EXAMPLE 3

Asymmetric hydrogenation of *mono*-methyl *mono*-*tert*-butyl 2(E)-phenethylidenesuccinate: Preparation of *mono*-methyl *mono*-*tert*-butyl 2(R)-phenethylsuccinate. A Fisher-Porter bottle was charged with mono-methyl mono-*tert*-butyl 2(E)-phenethylidenesuccinate (9.7g, 33 mmol) (Example 2) and rhodium (R,R) DiPAMP [(R₁R)-(1,2-ethanediyl bis[(O-methoxy-phenyl)phenylphosphine] (200mg, .265 mmol) and 30 mL of degassed methanol. The solution was then flushed with nitrogen 5 times and with hydrogen five times and pressurized with hydrogen to 40 psig. The solution was hydrogenated at room temperature for 20h and then the solvent removed on a rotary evaporator to give oil that was taken up in dichloromethane and passed through a short column of silica gel and concentrated to give 9.03g, 94% of pure product as an oil. ¹H NMR (CDCl₃) 300 MHz 7.35-7.17(m, 5H), 3.74(s, 3H), 2.88(m, 1H), 2.68(m, 3H), 2.45(dd, J=5.1, 15.6 Hz, 1H), 2.00(m, 1H), 1.84(m, 1H), 1.45(S, 9H).

### EXAMPLE 4

Saponification of *mono*-methyl *mono*-*t*-butyl 2(R)-phenethylsuccinate: Preparation of *mono*-*t*-butyl 3(R)-phenethylsuccinate. A 100 mL round bottomed flask equipped with nitrogen inlet and magnetic stir bar was charged with *mono*-methyl *mono*-*t*-butyl 2(R)-phenethylsuccinate (9.03g, 31 mmol) (Example 3), water (20mL), tetrahydrofuran (30 mL) and lithium hydroxide (1.30g, 31 mmol). This solution was stirred at room temperature over night (ca 20h) and then concentrated on a rotary evaporator. The residue was acidified with 1N KHSO₄ to pH=1 and extracted with ethyl acetate three times, the combined ethyl acetate extract was washed with brine, dried over MgSO₄, filtered and concentrated to give
4.43g, 51% of an oil that was taken directly onto the next step, ¹H NMR (CDCl₃) 300 MHz 7.35-7.18(m, 5H), 4.15(s, 1H), 2.90(m, 1H), 2.71(m, 2H), 2.48(dd, J=5.1, 15.6 Hz, 1H), 2.10(m, 1H), 1.89(m, 1H), 1.47(s, 9H).

### Example 5

Curtius rearrangement of *mono*-*t*-butyl 3(R)-phenethylsuccinate: Preparation of *t-butyl* 3(R)-[N-4-methoxybenzyloxycarbonyl]-amino-5-phenylpentanoate. A 250 mL three necked round bottomed flask equipped with a nitrogen inlet, reflux condenser, seran cap and magnetic stir bar was charged with *mono-t*-butyl 3(R)-phenethylsuccinate (4.43g, 0.016 mol) (Example 4), triethylamine (4.4 mL, 0.032 mol) and toluene (50 mL). The solution was warmed to 85°C. and then treated with diphenylphosphoryl azide (3.4 mL, 0.016 mol) via syringe over ca. 10m. After stirring at 85°C. for an additional 1.5h, the mixture was treated with a solution of 4-methoxybenzyl alcohol (2.0mL, 0.016 mol) in 10 mL of toluene all at once. This solution was stirred an additional 3h and then cooled to room temperature and diluted with an equal volume of ethyl acetate and poured into a separatory funnel. The solution was washed with 1N KHSO₄ 2 times, with saturated aqueous NaHCO₃ two times, with brine, dried over anhyd. MgSO₄, filtered and concentrated in vacuo. The residue was chromatographed on a Prep-500 eluting with hexane/ethyl acetate to afford a pure fraction that was concentrated and crystallized from hexane to give 1.91g, 29%, mp 57.7-59.4°C., an additional 1.61g was obtained by chilling the filtrate, for a combined yield of 53%. [α]_{D}=+ 9.6 (c=1, MeOH). ¹H NMR (acetone-D₆) 300 MHz 7.43-7.18(m, 7H), 6.96(d, J=8.4 Hz, 2H), 6.30(brd, 1H), 5.07(s, 2H), 4.06(m, 1H), 3.83(s, 3H), 2.73(m, 2H), 2.50(m, 2H), 1.89(m, 2H), 1.45(s, 9H).

### EXAMPLE 6

Stobbe condensation of phenylpropionaldehyde with dimethyl succinate: Preparation of methyl 2(E)-phenylpropylidene succinate dicyclohexylammonium salt. A 1000 mL 4-necked round bottomed flask equipped with mechanical stirrer, thermometer adapter, reflux condenser, nitrogen inlet and constant pressure addition funnel was charged with tert-butyl alcohol (300 mL) and potassium tert-butoxide (49.8g, 0.44 mol). [Note: the potassium tert-butoxide was added slowly to the tert-butyl alcohol with stirring to prevent clumping.] The solution was stirred while a solution of dimethyl succinate (74g, 0.5 mol) and phenylpropionaldehyde (53.6g, 0.4 mol) in tert-butyl alcohol (50 mL) was added from the dropping funnel over ca. 40m. The solution was warmed to an internal temperature of 63°C. and was then maintained at 70°C. for an additional 3h. The solution was then concentrated on a rotary evaporator to remove most of the solvent and then the thick oil was diluted with 3N HCl and extracted with ether three times. The combined ethereal layer was extracted with saturated aqueous sodium bicarbonate three times, the combined aqueous extract was then acidified to pH=1 with 3N HCl and re-extracted with ether. This combined extract was washed with brine, dried over MgSO₄, filtered and concentrated on a rotary evaporator to give a thick brown oil, 84.7g, 86%. The crude NMR showed that other isomer(s) were present in minor amounts, but by far the major product was the desired product. A 500 mL Erlenmeyer flask was charged with the crude product and with 200 mL of ether and then was treated with dicyclohexylamine (61.7g, 0.341 mol) dropwise with cooling in an ice bath at such a rate that the ether did not boil. After ca. 1h a thick white precipitate formed that made stirring with a magnetic stir bar impossible. The flask was swirled several times to facilitate mixing and then the product was isolated by filtration and the filter cake was washed with several portions of isooctane. The product was then air dried under an infrared lamp for ca. 1h to give 81.7g, 56% yield, mp 179.0-180.5°C. ¹H NMR (CDCl₃) 300 MHz 7.35-7.16(m, 5H), 6.86(T, J=7.3Hz, 1H), 3.72(s, 3H), 3.25(s, 2H), 2.88(tt, J=3.6,11.1 Hz, 2H), 2.78(t, J=7.9 Hz, 1H), 2.53(td, J=7.3, 7.9 Hz, 1H), 1,98(m, 4H), 1.77(m, 4H), 1.63(m, 2H), 1.40(m, 4H), 1.20(m, 6H); ¹³C NMR (CDCl₃) 175.97, 169.05, 141.84, 130.16, 128.91, 128.81, 126.54, 53.60, 51.97, 36.16, 35.38, 31.16, 30.10, 25.76, 25.41.

### EXAMPLE 7

Asymmetric reduction of methyl 2(E)-phenylpropylidene succinate dicyclohexylammonium salt: Preparation of methyl 2(R)[3-phenyl]propyl succinate dicyclohexylammonium salt. A large Fisher-Porter bottle equipped with a magnetic stir bar was charged with the olefin (12.65g, 29.5 mmol) (Example 6) and rhodium (R,R)-DiPAMP catalyst (233mg, .295 mmol). The bottle was evacuated and then degassed methanol (150 ML) was added via cannula. The solution was stirred and purged 5 times with nitrogen and then 5 times with hydrogen to a final pressure of 2000 Torr. The solution was hydrogenated for 16h and then the contents concentrated on a rotary evaporator to give a brown oil. The crude material was mixed in ethyl acetate with a little hexane and allowed to stand whereupon the pure product crystallized to give 10.5g, 82%, mp 81-83°C. [α]_{D} @ 23°C.= +8.8° (c=1.04, MeOH). ¹H NMR (CDCl₃) 300 MHz 7.29-7.10(m, 5H), 6.13(brs, 2H), 3.63(s, 3H), 2.87(m, 3H), 2.58(m, 3H), 2.27(dd, J=5.7, 15.9 Hz, 1H), 1.96(m, 4H), 1.75(m, 4H), 1.60(m, 6H), 1.36(m, 4H), 1.19(m, 6H).

### EXAMPLE 8

Esterification of methyl 2(R)[3-phenyl]propyl succinate with tert-butyl alcohol: Preparation of mono-methyl mono-t-butyl 2(R)[3-phenyl]propyl succinate. A sample of methyl 2(R)[3-phenyl]propyl succinate dicyclohexylammonium salt (3.53g, 8.2 mmol) (Example 7) was treated with 1N KHSO₄ (50 mL) and then extracted 3X with ethyl acetate. The combined ethyl acetate extract was washed with brine, dried over MgSO₄, filtered and concentrated on a rotary evaporator to give the free acid, 2.01g. Methyl 2(R)[3-phenyl]propyl succinate (2.01g, 8.0 mmol) was dissolved in dichloromethane (200 mL) and treated with t-butyl alcohol (2.3 mL, 24 mmol), dimethylaminopyridine (490mg, 4.0 mmol) and dimethylaminopyridine hydrochloride (130mg. 0.13 mmol). The solution was cooled to 0°C. in an ice bath and treated with dicyclohexylcarbodiimide (1.90g, 9.0 mmol). The solution was allowed to warm to room temperature and stirred for an additional 3h. The precipitated dicyclohexyl urea was removed by filtration and the filtrate washed with 0.2N HCl, sat. aq. NaHCO₃, brine, dried over MgSO₄, filtered and concentrated to give a brown oil 2.44g, ¹H NMR (CDCl₃) 300 MHz 7.30-7.10(m, 5H), 3.68(s, 3H), 2.80(m, 1H), 2.60(m, 3H), 2.32(dd, J=5.1, 15.6 Hz, 1H), 1.62(m, 4H), 1.42(s, 9H).

### EXAMPLE 9

Saponification of mono-methyl mono-t-butyl 2(R)[3-phenyl]propyl succinate: Preparation of mono-t-butyl (R)[3-phenyl]propyl succinate. The crude ester from the above reaction (2.44g, 8.0 mmol) (Example 8) was dissolved in a mixture of tetrahydrofuran (30 mL) and water (20 mL) and then treated with lithium hydroxide (340mg, 8.0 mmol) at room temperature for a period of 16h. The solution was concentrated on a rotary evaporator and then acidified with KHSO₄ and the aqueous phase extracted with ethyl acetate 3X, washed the ethyl acetate solution, with brine, dried over MgSO₄, filtered and concentrated on a rotary evaporator to give a slightly yellow oil, 1.48g, ¹H NMR (CDCl₃) 300 MHz 7.30-7.10(m, 5H ), 2.81(m, 1H), 2.60(m, 3H), 2.53(dd, J=5.1, 15.6 Hz, 1H), 1.69(m, 4H), 1.42(s, 9H).

### EXAMPLE 10

Curtius rearrangement of mono-t-butyl 3(R)[3-phenyl]propyl succinate: Preparation of t-butyl 3(R)-[N-Carbobenzyloxy]-amino-6-phenylhexanoate. A 100 mL round bottomed flask equipped with reflux condenser, nitrogen inlet and outlet, magnetic stir bar, thermometer adapter and serum cap was charged with mono-t-butyl 3(R)[3-phenyl]propyl succinate (1.48g, 5.1 mmol) (Example 9), triethylamine (1.4mL, 10.2 mmol, 2 equivalents) and toluene (20 mL). The solution was warmed to 85°C. and then treated with diphenylphosphoryl azide (1.1mL, 5.1 mmol) via syringe over ca. 5m. The solution was maintained at that temperature for an additional 120m and then treated with benzyl alcohol (0.53mL, 5.1 mmol) via syringe and then stirred at 85°C. for 3h. The solution was diluted with ethyl acetate and poured into a separatory funnel and washed with 1N KHSO₄, sat. aq. NaHCO₃, brine, dried over MgSO₄, filtered, and concentrated on a rotary evaporator to give a yellow oil that was purified by radial chromatography on SiO₂ eluting with hexane/ethyl acetate to give pure product 950mg, 47%. ¹H NMR (acetone-D₆) 300 MHz 7.45-7.18(m, 10H), 6.30(brd, 1H), 5.12(s, 2H), 4.10(m, 1H), 2.70(m, 2H), 2.48(d, J=7 Hz, 2H), 1.74(m, 2H), 1.63(m, 2H), 1.45(s, 9H).

### EXAMPLE 11

Cleavage of t-butyl 3(R)-[N-Carbobenzyloxy]-amino-6-phenylhexanoate with trifluoroacetic acid: Preparation of 3(R)-[N-Carbobenzyloxy]-amino-6-phenylhexanoic acid dicyclohexylammonium salt. A 50 mL round bottomed flask equipped with a reflux condenser, nitrogen inlet and magnetic stir bar was charged with t-butyl 3(R)-[N-Carbobenzyloxy]-amino-6-phenylhexanoate (950mg, 2.4 mmol) (Example 10), toluene (20 mL) and trifluoroacetic acid (20 mL). The solution was stirred at room temperature for 16h and then concentrated on a rotary evaporator. The residue was taken up in ethyl acetate and washed with sat. aq. NaHCO₃. The combined aqueous extract was acidified with 1N HCl and extracted 3X with ethyl acetate. The combined ethyl acetate extract was washed with brine, dried over MgSO₄, filtered and concentrated to give a clear oil that was taken up in ether and treated with dicyclohexylamine to afford the pure salt, 310 mg, 25%, mp 137-141°C., ¹H NMR (methanol-D₄) 300 MHz 7.41-7.11(m, 10H), 5.10(q, 2H), 4.00(m, 1H), 3.20(m, 2H), 2.63(m, 2H), 2.37(m, 2H), 2.10(m, 4H), 1.90(m, 4H), 1.71(m, 6H), 1.46(m, 10H).

### EXAMPLE 12

Preparation of mono-methyl itaconate. A 250 mL round bottomed flask equipped with a reflux condenser, nitrogen inlet and magnetic stir bar was charged with itaconic anhydride (22.4g, 0.2 mol) and 100 mL of anhydrous methanol. The solution was warmed to reflux for 80m and then the excess methanol was removed on a rotary evaporator to give a white solid. The solid was taken up in boiling iso-octane/methyl ethyl ketone and placed in a refrigerator whereupon white needles formed, 11.32g, 39% mp 70-72°C. two additional crops of white needles were produced after further refrigeration, 12.0g for a combined yield of 80%. ¹H NMR (CDCl₃) 300MHz 6.51(s, 1H), 5.88(s, 1H), 3.75(s, 3H), 3.39(s, 2H).

### EXAMPLE 13

Preparation of mono-benzyl mono-methyl itaconate. A 250 mL round bottomed flask equipped with constant pressure addition funnel, nitrogen inlet and magnetic stir bar was charged with mono-methyl itaconate (s10.0g, 69.4 mmol) (Example 12), toluene (50 mL), and 1,8-diazabicyclo[5.4.0]undec-7-ene (DBU) (10.56g, 69.4 mmol). The solution was stirred at room temperature and treated with a solution of benzyl bromide (11.88g, 69.4 mmol) in toluene (25 mL) dropwise over ca. 25m. The solution was stirred for an additional 70m and then the contents were poured into a separatory funnel and washed with 3N HCl, saturated aqueous NaHCO₃, brine, dried over MgSO₄, filtered and concentrated in vacuo, to give 15.73g, 92% of product. Thin layer chromatography on SiO₂ with 10:1 hexane:ethyl acetate showed three spots, the product at R_{f}=0.19 and others at R_{f}=0.33 and 0.43. All 15.7g was introduced onto a Prep-500 SiO₂ column and eluted with 100% hexane to give the pure product, 12.92g, 76% (oil) as the major peak after the impurity peaks had come off. ¹H NMR (CDCl₃) 300MHz 7.40(s, 5H), 6.42(s, 1H), 5.78(s, 1H), 5.25(s, 2H), 3.68(s, 3H), 3.40(s, 2H). ¹³C NMR 170.86, 165.73, 135.64, 133.61, 129.37, 129.06, 128.75, 128.61, 66.58, 51.79, 37.40.

### EXAMPLE 14

Heck arylation of mono-benzyl mono-methyl itaconate: Preparation of mono-benzyl mono-methyl 2(E)benzylidene succinate. A 250 mL Fisher-Porter bottle was charged with mono-benzyl mono-methyl itaconate (10.00g, 42.7 mmol) (Example 13), triethylamine (12.7g, 0.126 mol), palladium acetate (190mg, 0.86 mmol, 2.5 mol%), tri-o-tolylphosphine (523mg, 1.72 mmol) and iodobenzene (6.90g, 33.8 mmol). The bottle was sealed and flushed 5X with nitrogen to a pressure of 1000 Torr. The bottle was placed in an oil bath at 100°C. for 4h. The bottle was opened and the contents washed out with ethyl acetate and 1N KHSO₄. This material was filtered through a pad of celite and then poured into a separatory funnel and the phases separated. The aqueous phase was washed with ethyl acetate 2X and the combined ethyl acetate solution washed with brine, dried over MgSO₄, filtered and concentrated on a rotary evaporator to give 16.66g of a brown oil 103%. Thin layer chromatography revealed that there were 2 major spots, SiO₂ 4:1 hexane:ethyl acetate. The crude product was introduced onto a Prep-500 SiO₂ column and eluted first with 100% hexane which gave 2.08g of isomerized product [mono-benzyl mono-methyl citraconate], oil, ¹H NMR (CDCl₃) 300 MHz 7.41(s, 5H), 6.87(d, J=1.5Hz, 1H), 5.28(s, 2H), 3.80(s, 3H), 2.36(d, J=1.5Hz, 3H). ¹³C NMR (CDCL₃) 166.81, 166.19, 143.73, 135.41, 129.16, 128.96, 128.74, 127.18, 67.26, 51.62, 14.30.] and then with 20% ethyl acetate in hexane to give 7.63g, 56% of the desired product as an oil. ¹H NMR (CDCl₃) 300 MHz 8.00(s, 1H), 7.48-7.34(m, 10H), 5.32(s, 2H), 3.71(s, 3H), 3.60(s, 2H). ¹³C NMR (CDCl₃)171.46, 167.04, 142.35, 135.86, 134.81, 128.96, 128.89, 128.57, 128.48, 128.16, 128.10, 125.88, 66.84, 52.01, 33.46.

### EXAMPLE 15

Asymmetric reduction of mono-benzyl mono-methyl 2(E)-benzylidene succinate: Preparation of mono-benzyl mono-methyl 2(R)-benzyl succinate. A Fisher-Porter bottle was charged with mono-benzyl mono-methyl 2(E)-benzylidene succinate (5.40g, 17.4 mmol) (Example 14), rhodium (R,R) DiPAMP (500mg) and degassed ethyl acetate (30 mL). The bottle was flushed 5X with nitrogen and then 5X with hydrogen and pressurized with hydrogen to a final pressure of 2000 Torr and allowed to hydrogenate at room temperature for 36h. The bottle was opened and the contents concentrated on a rotary evaporator to give 5.43g, 100% of the desired product, ¹H NMR (CDCl₃) 300 MHz 7.44-7.13(m, 10H), 5.15(s, 2H), 3.64(s, 3H), 3.24(m, 1H), 3.11(dd, J=6.6, 13.6Hz, 1H), 2.83(dd, J=8.9, 13.6Hz, 1H), 2.74(dd, J=8.9, 16.8Hz, 1H), 2.47(dd, J=5.3, 16.8Hz, 1H).

### EXAMPLE 16

Hydrogenolysis of mono-benzyl mono-methyl 2(R)-benzyl succinate: Preparation of mono-methyl 3(R)-benzyl succinate. The crude mono-benzyl mono-methyl 2(R)-benzyl succinate from the asymmetric reduction (5.4g, 17.4 mmol) (Example 15) was placed in a Fisher-Porter bottle along with glacial acetic acid (20 mL) and 10% palladium on carbon (100mg). The bottle was flushed 3X with nitrogen and then 5X with hydrogen, pressurized with hydrogen to 2000 Torr, and hydrogenated at room temperature for 24h. The bottle was opened and the contents filtered through a pad of celite, the celite was washed with ethyl acetate and the combined filtrate was concentrated on a rotary evaporator to give 3.38g, 88% of a semi solid, ¹H NMR (CDCl₃) 300 MHz 7.48-7.20(m, 5H), 3.69(s, 3H), 3.19(m, 2H), 2.83(dd, J=10.5, 15.5Hz, 1H), 2.70(dd, J=8.9, 17.0Hz, 1H), 2.46(dd, J=4.5, 17.0Hz, 1H). A sample of the acid (460mg, 2.07 mmol) was dissolved in ether (15 mL) and treated with dicyclohexylamine (375mg, 2.07 mmol) and then placed in a refrigerator for 24h whereupon crystals of the dicyclohexylammonian salt formed that were isolated by filtration and air dried to give 680mg, 81%, mp 99-101°C., ¹H NMR (CDCl₃) 300 MHz 8.65(brs, 2H), 7.27(s, 5H), 3.61(s, 3H), 3.18(dd, J=5.7, 13.2Hz, 1H), 2.99(m, 1H), 2.91(tt, J=3.6, 10.2Hz, 2H), 2.74(dd, J=8.7, 13.2Hz, 1H), 2.65(dd, J=8.7, 15.9Hz, 1H), 2.31(dd, J=5.7, 15.9Hz, 1H), 2.00(m, 4H), 1.80(m, 4H), 1.65(m, 2H), 1.40(m, 4H), 1.22(m, 6H).

### EXAMPLE 17

Curtius rearrangement of mono-methyl 3(R)-benzyl succinate: Preparation of methyl N-Moz-homo-β-phenylalanine. A solution of mono-methyl 3(R)-benzyl succinate (1.04g, 4.7 mmol) (Example 16) and triethylamine (570mg, 5.64 mmol, 1.2 equivalents) in toluene (20 mL) was heated to 80-90°C. and treated with diphenylphosphoryl azide (1.29g, 4.7 mmol) via syringe over 10m. After stirring at that temperature for an additional 1.5h 4-methoxybenzyl alcohol (714mg, 5.17 mmol) was added via syringe all at once and the solution stirred for an additional 2h at 85°C. The reaction mixture was diluted with ethyl acetate and poured into a separatory funnel and washed with 1N KHSO₄, sat. aq. NaHCO₃, brine, dried over anhyd. MgSO₄, filtered, and concentrated to give 1.75g of crude product that was purified by radial chromatography on SiO₂ eluting with hexane/ethyl acetate, the first thing to elute was the desired product, 1.14g, 68% as an oil, ¹H NMR (CDCl₃) 300 MHz 7.37-7.17(m, 7H), 6.92(d, J=8.4Hz, 2H), 5.30(d, J=8.2Hz, 1H), 5.05(s, 2H), 4.26(m, 1H), 3.85(s, 3H), 3.71(s, 3H), 2.99(dd, J=5.7,16.0Hz, 1H), 2.88(dd, J=5.7, 16Hz, 1H), 2.58(dd, J=4.8, 13.2Hz, 1H), 2.52(dd, J=7.5, 13.2Hz, 1H).

### EXAMPLE 18

Saponification of methyl N-Moz-homo-β-phenylalanine: Preparation of N-Moz-homo-β-phenylalanine. A 50 mL round bottomed flask equipped with a reflux condenser, nitrogen inlet and magnetic stir bar was charged with methyl N-Moz-homo-β-phenylalanine (580mg, 1.62 mmol) (Example 17), methanol (30 mL), water (10 mL), and lithium hydroxide (68mg, 1.63 mmol). The solution was stirred at room temperature while the progress was monitored by thin layer chromotography on SiO₂ eluting with 3:1 hexane:ethyl acetate (R_{f} of the starting material was 0.40), after 120m only a trace of starting material remained. Allowed the solution to stir over night. The solution was diluted with water and poured into a separatory funnel and extracted with ether. The aqueous phase was acidified with 1N KHSO₄ to pH=1 and extracted with ethyl acetate 3X, the combined ethyl acetate extract was washed with brine, dried over MgSO₄, filtered, and concentrated to give a white solid that was taken up in boiling hexane plus a small amount of ethyl acetate and allowed to stand whereupon crystals of pure product formed that were isolated by filtration and air dried to give 270mg, 48% mp 110.5-111.5°C., and additional crop was obtained by chilling the filtrate in an ice bath, 170mg, for a combined yield of 79%. ¹H NMR (CDCl₃) 300 MHz 7.30-7.12(m, 7H), 6.87(d, J=8.4Hz, 2H), 5.22(brd, 1H), 5.00(s, 2H), 4.20(m, 1H), 3.79(s, 3H), 2.90(m, 2H), 2.55(m, 2H).

### EXAMPLE 19

Hydrogenolysis of N-Moz-homo-β-phenylalanine: Preparation of homo-β-phenylalanine. A Fisher-Porter bottle was charged with N-Moz-homo-β-phenylalanine (100mg, 0.29mmol) Example 18), glacial acetic acid (10 mL) and 20mg of 10% palladium an on carbon. The solution was hydrogenated at 2000 Torr for 16h and then filtered through celite and-the solvent removed on a rotary evaporator to give a white solid that was recrystallized from a mixture of boiling acetone/methanol to give 40mg, 77%, mp 212-213 (dec)°C., [α]_{D} @ 24°C.=-17.8° (c=0.733, H₂O). ¹H NMR (D₂O) 300 MHz 7.50-7.30(m, 5H), 3.80(m, 1H), 3.06(dd, J=6.9, 13.9Hz, 1H), 2.97(dd, J=8.1, 13.9Hz, 1H), 2.60(dd, J=5.5, 17.0Hz, 1H), 2.47(dd, J=8.1, 17.0Hz, 1H).

### EXAMPLE 20

Wittig condensation of phenylbutyraldehyde with 4-methyl-1-tert-butyl-2-(triphenylphosphoranylidene) succinate: Preparation of 4-methyl 1-tert-butyl 2(E)-phenbutyrlidenesuccinate. A 250 mL round bottomed flask equipped with a nitrogen inlet and magnetic stir bar was charged with phenylbutyraldehyde (3.95g, 26.7 mmol), 4-methyl-1-tert-butyl-2-(triphenylphosphoranylidene) succinate (11.94g, 26.7 mmol) and tetrahydrofuran (50mL). The solution was stirred at room temperature for 5 days and then concentrated in vacuo. The residue was taken up in ethyl acetate and the precipitated triphenylphosphine oxide removed by filtration and the filtrate concentrated and chromatographed on SiO₂ on a Prep-500 eluting with hexane/ethyl acetate to give 2.02g, 24% as an oil ¹H NMR (CDCl₃) 300 MHz 7.35-7.17(m, 5H), 6.92(t, J=7.8 Hz, 1H), 3.72(s, 3H), 3.30(s, 2H), 2.69(t, J=7.8 Hz, 2H), 2.23(q, J=7.8 Hz, 2H), 1.83(m, J=2H).

### EXAMPLE 21

Cleavage of mono-methyl mono-tert-butyl 2(E)-phenbutyrlidenesuccinate with trifluoroacetic acid: Preparation of 1-methyl 3(E)-phenbutyrlidenesuccinate. A 250 mL one necked round bottomed flask equipped with a nitrogen inlet and magnetic stir bar was charged with mono-methyl mono-tert-butyl 2(E)-phenbutyrlidenesuccinate (2.02g, 6.3 mmol) (Example 20), toluene (25 mL) and trifluoroacetic acid (25 mL). The mixture was stirred at room temperature for 45m and then the solution was concentrated on a rotary evaporator. The residue was dissolved in ethyl acetate and extracted with sat. aq. NaHCO₃ 3X. The combined aqueous extracts were acidified with IN HCl and extracted with ethyl acetate 3X. The combined ethyl acetate extracts were washed with brine, dried over anhyd. MgSO₄, filtered, and concentrated to give a light yellow oil, 0.88g, 53%, this material was used directly in the next step, ¹H NMR (methanol -D₄) 300 MHz 7.33-7.01(m, 5H), 7.04(t, J=7.8 Hz, 1H), 3.70(s, 3H), 3.33(s, 2H), 2.68(q, J=7.8 Hz, 2H), 2.24(q, J=7.8 Hz, 2H), 1.81(m, 2H).

### EXAMPLE 22

Asymmetric reduction of 1-methyl 3(E)-phenbutyrlidenesuccinate: Methyl 7-phenyl-3(R)-carboxy heptanoate. A small Fisher-Porter bottle was charged with 1-methyl 3(E)-phenbutyrlidenesuccinate (0.88g, 3.4 mmol) (Example 21), rhodium (R,R) DiPAMP catalyst (50mg, 0.066 mmol), triethylamine (0.47mL, 3.4 mmol) and degassed methanol (20 mL). The bottle was fluhed 5X with nitrogen and 5X with hydrogen and hydrogenated at 2000 Torr and room temperature for 20h. The bottle was opened and the solvent removed on a rotary evaporator to give a brown oil that was taken up in hot iso-octane and concentrated to give a clear oil, 500mg, 56% of material that was taken onto the next step. ¹H NMR (CDCl₃) 300 MHz 7.35-7.16(m, 5H), 3.71(s, 3H), 2.89(m, 1H), 2.75(dd, J=8, 16 Hz, 1H), 2.65(m, 2H), 2.48(dd, J=5, 16 Hz, 1H), 1.66(m, 4H), 1.44(m, 2H).

### EXAMPLE 23

Curtius rearrangement of methyl 7-phenyl-3(R)-carboxy heptanoate and saponification: Preparation of 3(R)-(N-4-methoxybenzyloxycarbonylamino)-7-phenyl heptanote. A 100 mL three necked round bottomed flask equipped with serum cap, nitrogen inlet, reflux condenser and magnetic stir bar was charged with methyl 7-phenyl-3(R)-carboxy heptanoate (500mg, 1.9 mmol) (Example 22), triethylamine (0.53mL, 3.8 mmol), and toluene (10 mL). The solution was warmed to 85°C. and then treated with diphenylphosphoryl azide (0.41mL, 1.9 mmol) via syringe over 15m. The solution was stirred at this temperature for 1h and then was treated with 4-methoxybenzyl alcohol (0.24mL, 1.9 mmol) via syringe. The solution was maintained at 85°C. for an additional 1.5h and then cooled to room temperature, diluted with ethyl acetate and poured into a separatory funnel. The solution was washed with 1N KHSO₄, sat. aq. NaHCO₃, brine, dried over anhyd. MgSO₄, filtered, and concentrated in vacuo. The crude product was taken up in THF (10 mL), and water (5 mL) and then treated with lithium hydroxide (110mg, 2.5 mmol. The solution was stirred at room temperature over night (18h) and then concentrated on a rotary evaporator. The residue was acidified with 1N KHSO₄ and extracted with ethyl acetate 3X, the combined extracts were washed with brine, dried over anhyd. MgSO₄, filtered, and concentrated to give a white solid, 123mg, 17%, mp 114-116°C. ¹H NMR (acetone-D₆) 300 MHz 7.32-7.10(m, 7H), 6.89(d, J=8.4 Hz, 2H), 6.65(brd, 1H), 4.95(s, 2H), 3.98(m, 1H), 3.77(s, 3H), 2.62-2.42(m, 4H), 1.60(m, 4H), 1.40(m, 2H).

### EXAMPLE 24

Preparation of 1,1-diphenyl-1,4-butanediol. A dry 500 mL three-necked round bottomed flask equipped with a reflux condenser, nitrogen inlet, addition funnel, magnetic stir bar and thermometer adapter was charged with gamma-butyrolactone (7.7 mL, 0.1 mol) and 150 mL of dry n-hexane. The solution was cooled in an ice bath and treated with a solution of phenyllithium in cyclohexane (2M, 155 mL, 0.31 mol) drop wise from the addition funnel over a period of 3 h. The solution was stirred at room temperature for a period of 16 h and then the solution poured onto crushed ice. The mixture was then poured into a separatory funnel and the phases separated. The aqueous phase was extracted with ethyl acetate (3X), the combined organic phase was washed with 1N hydrochloric acid, sat. aq. NaHCO₃, brine, dried over anhyd. MgSO₄, filtered, and concentrated to give a white solid, 16.41g, 68% of the desired product. ¹H NMR (CDCl₃) 300 MHz 7.55-7.20 (m, 10H), 3.67(t, 2H), 2.47(t, 2H), 1.62(m, 2H).

### EXAMPLE 25

Reduction of 1,1-diphenyl-1,4-butanediol with triethylsilane/ trifluoroacetic acid: Preparation of 4,4-diphenyl-1-butanol. A 100 mL round bottomed flask equipped with an addition funnel was charged with 1,1-diphenyl-1,4-butanediol (16.41g, 0.068 mol) (Example 24) and 20.9 mL (0.27 mol) of trifluoroacetic acid. To this stirring solution was added triethylsilane (24.0 mL, 0.15 mol) over 30 m from the addition funnel. The mixture was stirred for an additional 30 m and then concentrated and the residue treated with sat. aq. NaHCO₃. The aq. solution was extracted with ethyl acetate (3X), washed with brine, dried over anhyd. MgSO₄, filtered, and concentrated to give a yellow oil, (the oil was found to be a mixture of the desired alcohol and trifluoroacetate ester). The oil was then heated with 10% NaOH (50 mL) at reflux for 30 m, cooled to room temperature and extracted with ether (3X). The ethereal extracts were combined and washed with sat. aq. NaHCO₃, dried over anhyd. MgSO₄, filtered, and concentrated, to give an oil 6.65 g, 43%. ¹H NMR (CDCl₃) 300 MHz 7.45-7.15(m, 10H), 3.95(t, 1H), 3.66(t, 2H), 2.18(m, 2H), 1.58(m, 2H).

### EXAMPLE 26

Oxidation of 4,4-diphenyl-1-butanol with pyridinium chlorochromate: Preparation of 4,4-diphenyl butyraldehyde. A 250 mL three neck round bottomed flask equipped with reflux condenser, nitrogen inlet, mechanical stirrer, and serum cap was charged with pryidinium chlorochromate (10.78 g, 0.05 mol) and dichloromethane (100 mL). To this stirring solution was added a solution of 4,4-diphenyl-1-butanol (6.65 g, 0.025 mol) (Example 25) in dichloromethane (10 mL) via syringe. The solution was stirred at room temperature for 1 h and then the organic solution decanted from the black gummy residue. The residue was swirled twice with additional dichloromethane and the combined organic phase washed with sat. aq. NaHCO₃, dried over anhyd. MgSO₄, filtered and concentrated to give an oil that was purified by flash chromatography on SiO₂ eluting with hexane/ethyl acetate to give the desired aldehyde as an oil, 5.34g, 95%. ¹H NMR (CDCl₃) 300 MHz 9.75(s, 1H), 7.43-7.19(m, 10H), 3.96(t, 3H), 2.45(m, 4H).

### EXAMPLE 27

Wittig reaction of 4,4-diphenyl butyraldehyde: Preparation of Methyl 7,7-diphenyl-3-carbo-tert-butoxy-hept-3(E)-ene. A mixture of 1-tert-butyl-4-methyl-2-(triphenylphos-phoranylidene) succinate (18.36 g, 0.04 mol) (see e.g. Example 1) and 4,4-diphenyl butyraldehyde (9.19 g, 0.04 mol) (Example 26) in tetrahydrofuran (40 mL) was stirred under nitrogen at room temperature for 8 d. The solvent was removed and the residue taken up in n-hexane whereupon the triphenylphosphine oxide precipitated and was removed by filtration and the filterate concentrated and purified by chromatography on a Waters Prep-500 instrument eluting with 5% ethyl acetate in n-hexane to give 5.92g, 38% of pure product as an oil. ¹H NMR (CDCl₃) 300 MHz 7.356-7.20(m, 10H), 6.91(t, J=7.4Hz, 1H), 3.95(t, J=7.7Hz, 1H), 3.69(s, 3H). 3.17(s, 2H), 2.27-2.14(m, 2H), 1.51(s, 9H). FAB mass spectrum: MH⁺=395.

### EXAMPLE 28

Treatment of Methyl 7,7-diphenyl-3-carbo-tert-butoxy-hept-3(E)-ene with trifluoroacetic acid: Preparation of methyl 7,7-diphenyl-3-carboxy-hept-3(E)-eneoic acid. A solution of methyl 7,7-diphenyl-3-carbo-tert-butoxy-hept-3(E)-ene (5.92 g, 0.015 mol) (Example 27) and toluene (25 mL) was treated with trifluoroacetic acid (25 mL) at room temperature for 1.5 h and then concentrated.
The residue was taken up in ethyl acetate and washed several times with water, dried over anhyd. MgSO₄, filtered and concentrated to give a brown oil, 4.24g. The oil was taken up in warm hexane and allowed to stand whereupon white crystals of product formed, 2.74g, 54% ¹H NMR (CDCl₃) 300 MHz 7.30-7.17(m, 10H), 7.10(t, J=7.5Hz, 1H), 3.90(t, J=7.5Hz, 1H), 3.64(s, 3H), 3.17(s, 2H), 2.26-2.13(m, 4H). FAB mass spectrum (MH⁺) = 339.

### EXAMPLE 29

Asymmetric hydrogenation of methyl 7,7-diphenyl-3-carboxy-hept-3(E)-eneoic acid with rhodium (R,R)-DiPAMP: Preparation of methyl 7,7-diphenyl-3(R)-carboxy-heptanoic acid. A small Fisher-Porter bottle was charged with methyl 7,7-diphenyl-3-carboxy-hept-3(E)-eneoic acid (2.74 g, 8.1 mmol) (Example 28), rhodium (R,R) DiPAMP catalyst (50mg, 0.066 mmol), triethylamine (1.4mL, 10.2 mmol) and degassed methanol (20 mL). The bottle was flushed 5X with nitrogen and 5X with hydrogen and hydrogenated at 2000 Torr and room temperature for 20h. The bottle was opened and the solvent removed on a rotary evaporator to give a brown oil that was taken up in ethyl acetate, washed with 1N KHSO₄, dried over anhyd. MgSO₄, filtered and concentrated to give an oil that was purified by radial chromatography with 1:1 hexane: ethyl acetate (v:v) to provide 1.63g, 59% of desired product. ¹H NMR (CDCl₃) 300 MHz 7.30-7.14(m, 10H), 3.89(t, J=7.7Hz, 1H), 3.66(s, 3H), 2.83(m, 1H), 2.65(dd, J=9.0, 16.7 Hz, 1H), 2.38(dd, J=5.2, 16.7Hz, 1H), 2.06(m, 2H), 1.75(m, 1H), 1.59(m, 1H), 1.34(m, 2H). FAB mass spectrum (MH⁺)=341.

### EXAMPLE 30

Curtius rearrangement of methyl 7,7-diphenyl-3(R)-carboxy-heptanoic acid: Preparation of methyl 7,7-diphenyl-3(R)-(N-4-methoxybenzyloxy carbonyl)amino hexanoate. A 100 mL three necked round bottomed flask equipped with a nitrogen inlet, reflux condenser, serum cap and magnetic stir bar was charged with methyl 7,7-diphenyl-3(R)-carboxy-heptanoic acid (1.63g, 4.8 mmol) (Example 29), triethylamine (1.4 mL, 9.6 mmol) and toluene (15 mL). The solution was warmed to 85°C. and then treated with diphenylphosphoryl azide (1.03 mL, 4.8 mmol) via syringe over ca. 10m. After stirring at 85°C. for an additional 1.5h, the mixture was treated with a solution of 4-methoxybenzyl alcohol (0.6 mL, 4.8 mmol) in 1 mL of toluene all at once. This solution was stirred an additional 3h and then cooled to room temperature and diluted with an equal volume of ethyl acetate and poured into a separatory funnel. The solution was washed with 1N KHSO₄ 2X, NaHCO₃ 2X, brine, dried over anhyd. MgSO₄, filtered and concentrated in vacuo. The residue was purified by radial chromatography on SiO₂ eluting with 20% ethyl acetate in hexanes to give 1.49g, 65% of the desired product. ¹H NMR (acetone-D₆) 400 MHz 7.33-7.23(m, 10H), 7.14(d, J=8.4Hz, 2H), 6.88(d, J=8.4Hz, 2H), 6.08(brd, 1H), 4.95(s, 2H), 3.95(m, 2H), 3.80(s, 3H), 3.58(s, 3H), 2.50(dd, J=6.6,16.3Hz, 1H), 2.45,16.3Hz, 1H), 2.13(m,1H), 2.02(m, 1H), 1.60(m, 2H), 1.33(m, 2H). FAB mass spectrum (MH⁺)=476.

### EXAMPLE 31

Saponification of methyl 7,7-diphenyl-3(R)- (N-4-methoxybenzyloxy carbonyl)amino hexanoate: Preparation of 7,7-diphenyl-3(R)-(N-4-methoxybenzyloxy carbonyl)amino hexanoic acid dicyclohexylammonium salt. A 50 mL round bottomed flask equipped with a reflux condenser, nitrogen inlet and magnetic stir bar was charged with methyl 7,7-diphenyl-3(R)- (N-4-methoxybenzyloxy carbonyl)amino hexanoate (1.480 g, 3.1 mmol) (Example 30), tetrahydrofuran (20 mL), water (10 mL), and lithium hydroxide (260 mg, 6.2 mmol). The solution was stirred at room temperature while the progress was monitored by thin layer chromotography. After 0.5 h at room temperature the reaction was judged to be complete and the solvent was removed on a rotary evaporator. The residue was diluted with water and washed with ether and the aqueous solution acidified with 1N KHSO₄ to pH=1 and extracted with ethyl acetate (3X). The combined organic extract was dried over anhyd. MgSO₄, filtered and concentrated to give an oil that was taken up in ether and treated with dicyclohexyl amine (2.8 mL, 2.8 mmol) whereupon the product solidified and was isolated by filtration and air dried to give 1.18g, 59% mp 115-117°C. [α]_{D} @ 25 °C=-2.8° (c=1, MeOH), ¹H NMR (acetone-D₆) 400 MHz (of the free acid) 7.31-7.23(m, 10H), 7.13(d, J=8.5Hz, 2H), 6.90(d, J=8.5Hz, 2H), 6.10(brd, 1H), 4.95(s, 2H), 3.95(m, 2H), 3.79(s, 3H), 2.52(dd, J=6.2, 15.6Hz, 1H), 2.43(dd, J=6.2, 15.6Hz, 1H), 2.15(m, 1H), 2.04(m, 1H), 1.62(m, 2H), 1.34(m, 2H).

It is contemplated that carbamate-protected homo-β-amino acids containing other R' and R'' groups as defined above can be synthesized according to the described procedure with similar yields.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

## Claims

1. A method of preparing homo-β-amino acid of an optical purity sufficient to exhibit optical activity comprising reacting an isocyanate derivative of a 1-carboalkoxy-3-mono-substituted succinic acid of an optical purity sufficient to exhibit optical activity with an alcohol selected from the group consisting of primary and secondary alcohols to produce a carbamate-protected ester of an optical purity sufficient to exhibit optical activity, saponifying said ester to produce the corresponding carbamate-protected homo-β-amino acid of an optical purity sufficient to exhibit optical activity and deprotecting said carbamate-protected homo-β-amino acid.

2. The method of Claim 1 wherein the isocyanate derivative is obtained by effecting Curtius rearrangement of the 1-carboalkoxy-3-mono-substituted succinic acid.

3. The method of Claim 1 wherein the primary and secondary alcohols are represented by the formula R''OH wherein R'' represents alkyl, cycloalkyl, silyl, aryl, alkaryl and aralkyl radicals.

4. The method of Claim 3 wherein R'' represents alkyl radicals having from 1 to about 12 carbon atoms, cycloalkyl radicals having from about 4 to about 12 carbon atoms, and aryl radicals.

5. The method of Claim 1 wherein said primary and secondary alcohols are selected from the group consisting of benzyl alcohol, isopropyl alcohol, 4-methoxybenzyl alcohol, 2-trimethylsilylethanol, fluorenyl methanol and benzhydrol.

6. The method of Claim 1 wherein said alcohols are selected from the group consisting of benzyl alcohol and 4-methoxybenzyl alcohol.

7. A method of Claim 1 wherein said carbamate-protected homo-β-amino ester is represented by the formula: 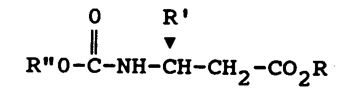 wherein R and R' independently represent alkyl, cycloalkyl, aryl, aralkyl and alkaryl radicals, and R'' represents radicals derived from said alcohol.

8. The method of preparing a carbamate-protected homo-β-amino ester of an optical purity sufficient to exhibit optical activity of the formula: 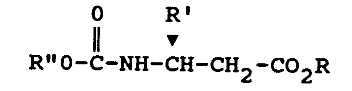 wherein R and R' independently represent alkyl radicals having from 1 to about 12 carbon atoms, cycloalkyl radicals having from about 4 to about 7 carbon atoms, and aryl, aralkyl and alkaryl radicals and R'' represents radicals derived from a primary or a secondary alcohol, said method comprising:
a) affecting Curtius rearrangement of a 1-carboalkoxy-3-mono-substituted succinic acid of an optical purity sufficient to exhibit optical activity to produce the corresponding optically active isocyanate derivative thereof; and
b) reacting the isocyanate derivative with a primary or secondary alcohol to produce the corresponding carbamate-protected homo-β-amino acid ester of an optical purity sufficient to exhibit optical activity.

9. A method of preparing a carbamate-protected homo-β-amino acid of an optical purity sufficient to exhibit optical activity of the formula: 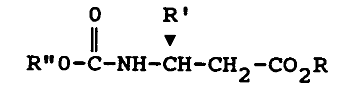 wherein R' represents alkyl radicals having from 1 to about 12 carbon atoms, cycloalkyl radicals having from about 4 to about 7 carbon atoms, and aryl, alkaryl and aralkyl radicals, and R'' represents radicals derived from a primary or a secondary alcohol, said method comprising saponifying the carbamate-protected homo-β-amino ester of Claim 8.

10. A method of preparing a homo-β-amino acid of an optical purity sufficient to exhibit optical activity of the formula: 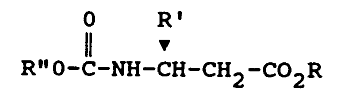 wherein R' represents alkyl radicals having from about 4 to about 7 carbon atoms, and aryl, aralkyl and alkaryl radicals, said method comprising deprotecting the carbamate-protected homo-β-amino acid of Claim 9.

11. Carbamate-protected homo-β-amino ester of an optical purity sufficient to exhibit optical activity of the formula: 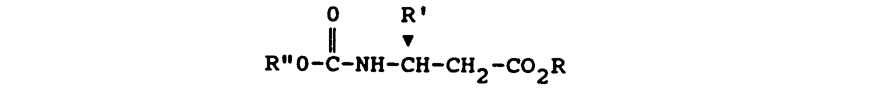 wherein R and R' independently represent alkyl radicals having from 1 to 12 carbon atoms, cycloalkyl radicals having from about 4 to about 7 carbon atoms, and aryl, alkaryl and aralkyl radicals and R'' represents radicals derived from a primary or a secondary alcohol.

12. Homo-β-amino estomer of Claim 11 wherein said R'' represents substituted and unsubstituted alkyl radicals having from 1 to about 12 carbon atoms, substituted and unsubstituted cycloalkyl radicals having from about 4 to about 7 carbon atoms, and substituted and unsubstituted silyl, aryl, alkaryl and aralkyl radicals.

13. Homo-β-amino ester of Claim 11 wherein said R'' represents a radical derived from an alcohol selected from the group consisting of benzyl alcohol, isopropyl alcohol, 4-methoxybenzyl alcohol, 2-trimethylsilyl-ethanol, fluorenyl methanol and benzhydrol.

14. Homo-β-amino ester of Claim 11 wherein said R'' represents radicals derived from a primary alcohol.

15. Homo-β-amino ester of Claim 14 wherein said primary alcohol is selected from the group consisting of benzyl alcohol and 4-methoxybenzyl alcohol.

16. Carbamate-protected homo-β-amino acid of an optical purity sufficient to exhibit optical activity represented by the formula: 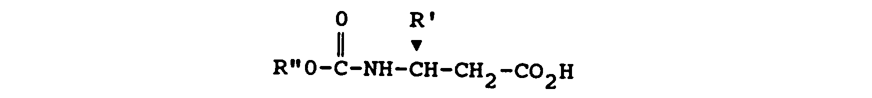 wherein R' represents substituted and unsubstituted alkyl radicals having from 1 to about 12 carbon atoms, substituted and unsubstituted cycloalkyl radicals having from about 4 to about 7 carbon atoms, and substituted and unsubstituted aryl, aralkyl and alkaryl radicals, and R'' represents radicals derived from a primary or a secondary alcohol.

17. Homo-β-amino acid of Claim 16 wherein said R'' represents a radical derived from an alcohol selected from the group consisting of benzyl alcohol, isopropyl alcohol, 4-methoxybenzyl alcohol, 2-trimethyl silylethanol, fluorenyl methanol and benzhydrol.

18. Homo-β-amino acid of Claim 16 wherein said R'' represents radicals derived from a primary alcohol.

19. Homo-β-amino acid of Claim 18 wherein said primary alcohol is selected from the group consisting of benzyl alcohol and 4-methoxybenzyl alcohol.

20. In a method for preparing homo-β-amino acids of an optical purity sufficient to exhibit optical activity wherein an isocyanate derivative of an amino acid ester is reacted with a tertiary alcohol to produce a carbamate-protected homo-β-amino ester which is subsequently saponified and then deprotected, the improvement which comprises reacting an isocyanate derivative of 1-carboalkoxy-3-mono-substituted succinic acid of an optical purity sufficient to exhibit optical activity with an alcohol selected from the group consisting of primary and secondary alcohols represented by the formula R''OH wherein R'' represents alkyl radicals having from 1 to about 12 carbon atoms, cycloalkyl radicals having from about 4 to about 7 carbon atoms and silyl, aryl, alkaryl and aralkyl radicals.
